# EUROPEAN PATENT APPLICATION

(11) **EP 4 292 437 A1**
(43) Date of publication of application: **20.12.2023**
(21) Application number: 22752849.4
(22) Date of filing: 14.02.2022
(51) Int. Cl.: A23C 19/082, A23C 20/02

(54) **METHODS RESPECTIVELY FOR PRODUCING CHEESE AND CHEESE ANALOGUE USING ENZYME**

(30) Priority: 15.02.2021 JP 2021022134
(71) Applicant: AJINOMOTO CO., INC., Chuo-ku Tokyo 104-8315 (JP)
(72) Inventor: HOMMA, Keisuke, Tokyo 108-0074 (JP); MINAMI, Mai, Tokyo 108-0074 (JP); FUJIMURA, Yuko, Kawasaki-shi, Kanagawa 210-8681 (JP)
(74) Representative: Strehl Schübel-Hopf & Partner
(86) International application number: PCT/JP2022/005540
(87) International publication number: WO 2022/173027

(57) **Abstract**

Production methods of cheese analogues improved in meltability and/or stretchability during heating and methods for suppressing the decrease are provided. Production methods of cheese analogues improved in meltability and/or stretchability during heating and methods for improving the meltability and/or stretchability are provided.

A method for producing cheese that shows a suppressed decrease in texture and/or stretchability of cheese due to heating, including allowing glucose oxidase, α-glucosidase, transglutaminase, or a combination of such enzymes to act on raw cheese, a method for suppressing a decrease in texture and/or stretchability of cheese due to heating, and an enzyme preparation to be used for the methods. A method for producing cheese analogues improved in meltability and/or stretchability during heating, including allowing α-glucosidase to act on a mixture containing plant-derived oil and starch, or starch before mixing with plant-derived oil, and a method for improving meltability and/or stretchability of cheese analogues during heating, and an enzyme preparation to be used for the methods.

## Description

### [Technical Field]

The present invention relates to methods for producing cheese that shows a suppressed decrease in quality (decrease in texture and/or stretchability) due to heating, methods for suppressing a decrease in quality of cheese (decrease in texture and/or stretchability) due to heating, and enzyme preparations to be used in these methods.

The present invention also relates to production methods of cheese analogues improved in meltability and/or stretchability during heating, methods for improving meltability and/or stretchability of cheese analogues during heating, and enzyme preparations to be used in these methods.

### [Background Art]

In convenience stores, supermarkets, and the like, steamed buns and steamed bread made by wrapping fillings in bun dough or bread dough and steaming them (e.g., Chinese steamed bun, steamed meat bun, steamed bean-paste bun, steamed carry bun, steamed pizza bun, steamed cheese meat bun, etc.) are preheated with a steamer and kept warm until purchased by the consumers in order to provide consumers with hot food that can be eaten immediately after purchase. Pizza buns and cheese meat buns containing cheese as filling have a problem that the qualities that affect the deliciousness of cheese (the original desirable texture of cheese (softness, smoothness on the tongue), stretchability (elasticity, elongation)) are deteriorated while they are heated and kept warm in a steamer.

Therefore, the development of cheese in which a decrease in quality (decrease in texture and/or stretchability) due to heating is suppressed is desired.

Patent Literature 1 discloses a method for modifying cheese, which includes adding transglutaminase and protease derived from filamentous fungi during the cheese production step.

Patent Literature 2 discloses a method for producing cheese, including treating a food product raw material containing milk protein with transglutaminase and calcium salt, in which the food product raw material contains milk protein that has been heat-treated at not less than 80°C.

Patent Literature 3 discloses a method for producing cheese, including a step of providing a first raw material liquid containing casein, a step of providing a second raw material liquid, a step of treating the first raw material liquid with a protein crosslinking enzyme to provide an enzyme-treated raw material liquid, a step of mixing the enzyme-treated raw material liquid with a second raw material liquid to provide cheese milk, and a step of processing the cheese milk using a coagulant into cheese.

The above-mentioned Literatures do not describe the problem of quality decrease that occurs when the produced cheese is heated and kept warm and no solution has been considered. In addition, all of the above-mentioned Literatures relate to methods for producing cheese by adding enzymes to raw materials before forming cheese. They do not disclose having enzymes act on cheese itself, unlike the present invention described later.

Cheese analogs (also referred to as analog cheeses, imitation cheeses, etc.) are foods that have been processed to have the appearance and texture of cheese as cheese substitute (e.g., food products in which fats and proteins in cheese are partially or entirely replaced with plant-derived components). In recent years, the market has been expanding due to rising awareness of animal welfare and health consciousness.

From the aspects of cooking aptitude, texture, and the like, cheese analogues, like cheese, preferably have good meltability and good stretchability when heated. However, conventional cheese analogues are not sufficiently satisfactory in meltability and stretchability during heating, and improvements are desired.

Patent Literature 4 discloses a food product containing plant proteins which is produced using transglutaminase in the production step. It is disclosed that the food product may further contain a protease. However, this Literature does not disclose action of α-glucosidase during the production of the cheese analogue.

### [Citation List]

### [Patent Literature]

[PTL 1]
   WO 2018/151197
[PTL 2]
   WO 2016/136906
[PTL 3]
   JP-A-2017-148054
[PTL 4]
   US-B-2017/0150734

### [Summary of Invention]

### [Technical Problem]

The present invention aims to provide production methods of cheese that shows a suppressed decrease in texture and/or stretchability of cheese due to heating, methods for suppressing a decrease in texture and/or stretchability of cheese due to heating, and enzyme preparations to be used in these methods.

The present invention also aims to provide production methods of cheese analogues improved in meltability and/or stretchability during heating, methods for improving meltability and/or stretchability of cheese analogues during heating, and enzyme preparations to be used in these methods.

### [Solution to Problem]

The present inventors have conducted intensive studies in an attempt to solve the aforementioned problems and found that cheese with suppressed decrease in the texture and/or stretchability due to heating can be produced by allowing the below-mentioned specific enzymes to act on the cheese. Based on the finding, the present inventors further conducted, intensive studies and completed the present invention (the first invention described below).

The present inventors have conducted intensive studies in an attempt to solve the aforementioned problems and found that cheese analogues improved in meltability and/or stretchability during heating can be produced by allowing α-glucosidase to act during production of cheese analogues. Based on the finding, the present inventors further conducted, intensive studies and completed the present invention (the second invention described below).

That is, the present invention provides the following [1] to [9] (to be referred to as the first invention in the present specification).
[1] A method for producing cheese that shows a suppressed decrease in texture and/or stretchability of cheese due to heating, comprising allowing an enzyme of any of the following (1) to (7):
   (1) glucose oxidase
   (2) α-glucosidase
   (3) transglutaminase
   (4) glucose oxidase and α-glucosidase
   (5) glucose oxidase and transglutaminase
   (6) α-glucosidase and transglutaminase
   (7) glucose oxidase, α-glucosidase and transglutaminase to act on target cheese to be acted on by the enzyme.
[2] The production method of the above-mentioned [1], wherein the aforementioned enzyme is allowed to act on a mixture of starch and the target cheese to be acted on by the enzyme.
[3] The production method of the above-mentioned [1] or [2], wherein the target cheese to be acted on by the enzyme is selected from the group consisting of mozzarella cheese, Gouda cheese, Cheddar cheese, and a combination of these.
[4] A method for suppressing a decrease in texture and/or stretchability of cheese due to heating, comprising allowing an enzyme of any of the following (1) to (7):
   (1) glucose oxidase
   (2) α-glucosidase
   (3) transglutaminase
   (4) glucose oxidase and α-glucosidase
   (5) glucose oxidase and transglutaminase
   (6) α-glucosidase and transglutaminase
   (7) glucose oxidase, α-glucosidase and transglutaminase
   to act on target cheese to be acted on by the enzyme.
[5] The suppressing method of the above-mentioned [4], wherein the aforementioned enzyme is allowed to act on a mixture of starch and the target cheese to be acted on by the enzyme.
[6] The suppressing method of the above-mentioned [4] or [5], wherein the target cheese to be acted on by the enzyme is selected from the group consisting of mozzarella cheese, Gouda cheese, Cheddar cheese, and a combination of these.
[7] An enzyme preparation for suppressing a decrease in texture and/or stretchability of cheese due to heating, comprising an enzyme of any of the following (1) to (7):
   (1) glucose oxidase
   (2) α-glucosidase
   (3) transglutaminase
   (4) glucose oxidase and α-glucosidase
   (5) glucose oxidase and transglutaminase
   (6) α-glucosidase and transglutaminase
   (7) glucose oxidase, α-glucosidase and transglutaminase, wherein the preparation is allowed to act on target cheese to be acted on by the enzyme.
[8] The enzyme preparation of the above-mentioned [7] for use in allowing the aforementioned enzyme to act on a mixture of starch and the target cheese to be acted on by the enzyme.
[9] The enzyme preparation of the above-mentioned [7] or [8], wherein the target cheese to be acted on by the enzyme is selected from the group consisting of mozzarella cheese, Gouda cheese, Cheddar cheese, and a combination of these.
   In addition, the present invention provides the following [10] to [18] (to be referred to as the second invention in the present specification).
[10] A method for producing a cheese analogue improved in meltability and/or stretchability during heating, comprising
   (1) a step of allowing α-glucosidase to act on a mixture of starch and a plant-derived oil, or
   (2) a step of allowing α-glucosidase to act on starch and mixing the obtained reaction product with a plant-derived oil.
[11] The production method of the above-mentioned [10], wherein the aforementioned (1) is a step of allowing α-glucosidase to act on a mixture of starch, a plant-derived oil, and a polysaccharide thickener, and
   the aforementioned (2) is a step of allowing α-glucosidase to act on starch, and mixing the obtained reaction product with a plant-derived oil and a polysaccharide thickener.
[12] The production method of the above-mentioned [10], wherein the aforementioned (1) is a step of allowing α-glucosidase to act on a mixture of starch, a plant-derived oil, and a vegetable protein, and
   the aforementioned (2) is a step of allowing α-glucosidase to act on starch, and mixing the obtained reaction product with a plant-derived oil and a vegetable protein.
[13] A method for improving meltability and/or stretchability of a cheese analogue during heating, comprising, during production of the cheese analogue,
   (1) a step of allowing α-glucosidase to act on a mixture of starch and a plant-derived oil, or
   (2) a step of allowing α-glucosidase to act on starch, and mixing the obtained reaction product with a plant-derived oil.
[14] The improving method of the above-mentioned [13], wherein the aforementioned (1) is a step of allowing α-glucosidase to act on a mixture of starch, a plant-derived oil, and a polysaccharide thickener, and
   the aforementioned (2) is a step of allowing α-glucosidase to act on starch, and mixing the obtained reaction product with a plant-derived oil and a polysaccharide thickener.
[15] The improving method of the above-mentioned [13], wherein the aforementioned (1) is a step of allowing α-glucosidase to act on a mixture of starch, a plant-derived oil, and a vegetable protein, and
   the aforementioned (2) is a step of allowing α-glucosidase to act on starch, and mixing the obtained reaction product with a plant-derived oil and a vegetable protein.
[16] An enzyme preparation for improving meltability and/or stretchability of a cheese analogue during heating, comprising
   (1) α-glucosidase to be allowed to act on a mixture of starch and a plant-derived oil during production of the cheese analogue, or
   (2) α-glucosidase to be allowed to act on starch before mixing with a plant-derived oil during production of the cheese analogue.
[17] The enzyme preparation of the above-mentioned [16],
   wherein the aforementioned (1) is α-glucosidase to be allowed to act on a mixture of starch, a plant-derived oil, and a polysaccharide thickener during production of the cheese analogue, and
   the aforementioned (2) is α-glucosidase to be allowed to act on starch before mixing a plant-derived oil and a polysaccharide thickener during production of the cheese analogue.
[18] The enzyme preparation of the above-mentioned [16],
   wherein the aforementioned (1) is α-glucosidase to be allowed to act on a mixture of starch, a plant-derived oil, and a vegetable protein during production of the cheese analogue, and the aforementioned (2) is α-glucosidase to be allowed to act on starch before mixing a plant-derived oil and a vegetable protein during production of the cheese analogue.

### [Advantageous Effects of Invention]

According to the present invention, cheese with suppressed decrease in the texture and/or stretchability due to heating can be produced by allowing the specific enzyme in the present invention to act on the target cheese to be acted on by the enzyme.

According to the present invention, cheese analogues improved in meltability and/or stretchability during heating can be produced by allowing α-glucosidase to act on starch or a mixture of starch and a plant-derived oil as a raw material during the production of the cheese analogues.

### [Brief Description of Drawings]

Fig. 1 is a photograph showing meltability of the cheese analogues of Comparative Examples and Examples by observation during heating in Experimental Example 3.
Fig. 2 is a photograph showing stretchability of the cheese analogues of Comparative Examples and Examples by observation during heating in Experimental Example 3.

### [Description of Embodiments]

### 1. The first invention

The present invention (the first invention) is explained in detail below.

Glucose oxidase (EC1.1.3.4) to be used in the present invention is an oxidase that catalyzes the reaction that produces gluconic acid and hydrogen peroxide using glucose, oxygen, and water as substrates. The hydrogen peroxide produced by this reaction promotes the formation of SS bond (disulfide bond) by oxidizing SH groups in a protein, and forms a crosslinked structure in the protein. Glucose oxidases of various origins are known such as those derived from microorganism, plant, and the like. The enzyme used in the present invention may be any enzyme as long as it has the aforementioned activity and its origin is not limited. It may also be a recombinant enzyme.

In the present invention, the activity unit of the glucose oxidase can be measured and defined as follows.

Using glucose as a substrate, glucose oxidase is reacted in the presence of oxygen to produce.hydrogen peroxide. The produced hydrogen peroxide is reacted with peroxidase in the presence of aminoantipyrine and phenol. The color tone exhibited by the produced quinoneimine dye is measured and quantified at a wavelength of 500 nm. An enzyme amount necessary for oxidizing 1 pmol of glucose in 1 min is defined as 1U (unit). The glucose oxidase to be used in the present invention may be a commercially available product, and one example is glucose oxidase derived from the microorganism commercially available from Nippon Chemical Industrial Co., Ltd. under the trade name "Sumizyme PGO". A glucose oxidase preparation containing catalase is commercially available, and the glucose oxidase used in the present invention may be a mixture with other enzyme as long as it has glucose oxidase activity.

The α-glucosidase (EC 3.2.1.20) to be used in the present invention is an enzyme that produces α-glucose by hydrolyzing a nonreducing terminal α-1,4-glucosidic bond. Among such α-glucosidases, transglucosidase is preferred. Incidentally, an enzyme commercially available from Amano Enzyme, Inc. under a trade name of "transglucosidase "Amano"" is one example of the α-glucosidase.

With respect to the enzyme activity of the α-glucosidase, the amount of the enzyme which produces 1 µg of glucose in 2.5 mL of a reaction mixture was defined as 1 U (unit) when 1 mL of 0.02 M acetate buffer (pH 5.0) was added to 1 mL of 1 mM α-methyl-D-glucoside, and 0.5 mL of an enzyme solution was added thereto to act at 40°C for 60 minutes.

Transglutaminase to be used in the present invention is an enzyme having an activity of catalyzing an acylmetastasis reaction using a glutamine residue in a protein or peptide as a donor and a lysine residue as a receptor. Transglutaminases of various origins are known, such as those derived from mammals, those derived from fish, those derived from microorganisms, and the like. The origin of the transglutaminase to be used in the present invention is not particularly limited as long as it has the above-mentioned activity, and any transglutaminase of any origin can be used. Alternatively, a recombinant enzyme may also be used. The transglutaminase to be used in the present invention may be a commercially available product. As a specific example, transglutaminase derived from microorganism which is commercially available from Ajinomoto Co., Inc. under the trade name of "Activa" TG can be used alone or in combination.

For the enzyme activity transglutaminase in the present invention, transglutaminase is reacted in a reaction system with benzyloxycarbonyl-L-glutamylglycine and hydroxylamine as substrates in a tris buffer at 37°C, pH 6.0, an iron complex of the produced hydroxamic acid is formed in the presence of trichloroacetic acid, the absorbance at 525 nm is measured, the amount of the hydroxamic acid is determined from the calibration curve, and the enzyme amount necessary for producing 1 pmol of hydroxamic acid in 1 min is defined as 1 unit (1U) (see JP-A-64-27471).

The method for producing cheese that shows a suppressed decrease in texture and/or stretchability of cheese due to heating of the present invention is characterized in that an enzyme of any of (1) glucose oxidase (sometimes to be referred to as enzyme (1) in the present specification), (2) α-glucosidase (sometimes to be referred to as enzyme (2) in the present specification), (3) transglutaminase (sometimes to be referred to as enzyme (3) in the present specification), (4) glucose oxidase and α-glucosidase (sometimes to be referred to as enzyme (4) in the present specification), (5) glucose oxidase and transglutaminase (sometimes to be referred to as enzyme (5) in the present specification) (6) α-glucosidase and transglutaminase (sometimes to be referred to as enzyme (6) in the present specification), and (7) glucose oxidase, α-glucosidase, and transglutaminase (sometimes to be referred to as enzyme (7) in the present specification) is allowed to act on the target cheese to be acted on by the enzyme.

Among these, enzyme (4) and enzyme (7) are preferred.

In the present invention, the "target cheese to be acted on by the enzyme" (hereinafter also to be referred to as raw cheese) is natural cheese obtained by removing whey from the milk produced by coagulating fresh milk as a raw material by using lactobacillus and rennet, and solidifying the milk.

In the present invention, the raw cheese shows, for example, a weight ratio of protein and fat (protein:fat) in the raw cheese of, for example, 1:0.1 - 5, preferably, 1:0.5 - 2.0.

In the present invention, specific examples of raw cheese include mozzarella cheese, Gouda cheese, Cheddar cheese, Edam cheese, raclette cheese, Parmigiano cheese, Camembert cheese, steppen cheese, Samsoe cheese, Monterey Jack, Emmentaler cheese, and combinations of these. One kind of raw cheese may be used or two or more kinds thereof may be used in combination. Commercially available cheese may be used and, for example, trade name "mozzarella melty cheese", trade name "NC shred cheese RKB" (both manufactured by QBB), and the like can be mentioned.

In the production method of the present invention, an enzyme is added to raw cheese and allowed to act thereon (enzyme reaction). When multiple enzymes are used in combination, the order of adding the enzymes to cheese does not matter particularly. Either one may be added first, and then the remaining enzymes may be added. It is preferable to add multiple enzymes simultaneously. Furthermore, raw materials generally used for food products may also be used in combination.

### enzyme (1): glucose oxidase

When enzyme (1) is used in the present invention, the amount of glucose oxidase to be added is such that the enzyme activity per 1 g of raw cheese is preferably not less than 0.001 U, more preferably 0.001 - 500 U, further preferably 0.03 - 210 U.

### enzyme (2): α-glucosidase

When enzyme (2) is used in the present invention, the amount of α-glucosidase to be added is such that the enzyme activity per 1 g of raw cheese is preferably not less than 0.03 U, more preferably 0.03 - 300000 U, further preferably 15 - 150000 U.

### enzyme (3): transglutaminase

When enzyme (3) is used in the present invention, the amount of transglutaminase to be added is such that the enzyme activity per 1 g of raw cheese is preferably not less than 0.0001 U, more preferably 0.0001 - 120 U, further preferably 0.05 - 12 U.

### enzyme (4): glucose oxidase and α-glucosidase

When enzyme (4) is used in the present invention, the amount of glucose oxidase to be added is such that the enzyme activity per 1 g of raw cheese is preferably not less than 0.001 U, more preferably 0.001 - 500 U, further preferably 0.03 - 210 U.

When enzyme (4) is used in the present invention, the amount of α-glucosidase to be added is such that the enzyme activity per 1 g of raw cheese is preferably not less than 0.03 U, more preferably 0.03 - 300000 U, further preferably 15 - 150000 U.

In addition, the ratio of the amounts of glucose oxidase and α-glucosidase to be added (glucose oxidase:α-glucosidase) is preferably 1 U:0.01 - 40000 U, more preferably 1 U:0.4 - 4000 U.

### enzyme (5): glucose oxidase and transglutaminase

When enzyme (5) is used in the present invention, the amount of glucose oxidase to be added is such that the enzyme activity per 1 g of raw cheese is preferably not less than 0.001 U, more preferably 0.001 - 500 U, further preferably 0.03 - 210 U.

When enzyme (5) is used in the present invention, the amount of transglutaminase to be added is such that the enzyme activity per 1 g of raw cheese is preferably not less than 0.0001 U, more preferably 0.0001 - 120 U, further preferably 0.05 - 12 U.

In addition, the ratio of the amounts of glucose oxidase and transglutaminase to be added (glucose
oxidase:transglutaminase) is preferably 1 U:0.00003 - 100 U, more preferably 1 U:0.001 - 10 U.

enzyme (6): α-glucosidase and transglutaminase

When enzyme (6) is used in the present invention, the amount of α-glucosidase to be added is such that the enzyme activity per 1 g of raw cheese is preferably not less than 0.03 U, more preferably 0.03 - 300000 U, further preferably 15 - 150000 U.

When enzyme (6) is used in the present invention, the amount of transglutaminase to be added is such that the enzyme activity per 1 g of raw cheese is preferably not less than 0.0001 U, more preferably 0.0001 - 120 U, further preferably 0.05 - 12 U.

In addition, the ratio of the amounts of α-glucosidase and transglutaminase to be added (α-glucosidase:transglutaminase) is preferably 1 U:0.000001 - 3 U, more preferably 1 U:0.00003 - 0.3 U.

### enzyme (7): glucose oxidase, α-glucosidase, and transglutaminase

When enzyme (7) is used in the present invention, the amount of glucose oxidase to be added is such that the enzyme activity per 1 g of raw cheese is preferably not less than 0.001 U, more preferably 0.001 - 500 U, further preferably 0.03 - 210 U.

When enzyme (7) is used in the present invention, the amount of α-glucosidase to be added is such that the enzyme activity per 1 g of raw cheese is preferably not less than 0.03 U, more preferably 0.03 - 300000 U, further preferably 15 - 150000 U.

When enzyme (7) is used in the present invention, the amount of transglutaminase to be added is such that the enzyme activity per 1 g of raw cheese is preferably not less than 0.0001 U, more preferably 0.0001 - 120 U, further preferably 0.05 - 12 U.

In addition, the ratio of the amounts of glucose oxidase, α-glucosidase, and transglutaminase to be added (glucose oxidase:α-glucosidase:transglutaminase) is preferably 1 U:0.01 - 40000 U:0.00003 - 100 U, more preferably 1 U:0.4 - 4000 U:0.001 - 10 U.

As the enzyme in the present invention, commercially available products such as formulations, seasonings, texture improvers, and the like containing the above-mentioned enzymes (for example, trade name "Okome fukkura chouriryo tsubukan up" (manufactured by Ajinomoto Co., Inc.) containing glucose oxidase, α-glucosidase, and transglutaminase) can also be used.

The reaction time of each enzyme is not particularly limited as long as the enzyme can act on the substrate substances, fat and protein (fat, protein, and starch when the below-mentioned starch is added), in cheese. As a realistic action time, 0.015 to 24 hr is preferred. Also, the reaction temperature is not particularly limited as long as the enzyme maintains its activity. As a realistic temperature, action at 5 to 60°C is preferred.

In the production method of the present invention, the enzyme is preferably added to the mixture containing raw cheese and starch and allowed to act. By containing starch, the substrates on which glucose oxidase and α-glucosidase act can be increased. In the present invention, modified starch (e.g., hydroxypropyl distarch phosphate) can contribute to the improvement of heat meltability and suppression of oil separation.

Examples of the starch to be used in the present invention include modified starches such as hydroxypropyl distarch phosphate, acetylated distarch adipate, acetylated distarch phosphate, acetylated oxidized starch, starch sodium octenylsuccinate, starch acetate, oxidized starch, hydroxypropyl starch, phosphated distarch phosphate, monostarch phosphate, distarch phosphate and the like; corn starch, wheat flour starch, rice starch, potato starch, tapioca starch, and sago starch. The modified starch is preferred and hydroxypropyl distarch phosphate is more preferred. Only one kind of starch may be used, or two or more kinds thereof may be used in combination.

In the present invention, when a mixture containing raw cheese and starch is reacted with an enzyme, the amount of starch to be added is preferably 0.1 to 20 g, more preferably 0.5 to 10 g, per 100 g of raw cheese.

By the production method of the present invention described above, it is possible to produce cheese that shows a suppressed decrease in texture and/or stretchability of cheese due to heating.

In the present specification, the method, temperature, and time of "heating" in the "decrease in texture and/or stretchability of cheese due to heating" are not particularly limited. For example, a method, temperature, and time of heating that cause a decrease in texture and/or stretchability when enzyme-free cheese is heated can be mentioned.

The temperature and time of the "heating" are, for example, not less than 65°C (or 65°C - 180°C) for 15 min (or 15 min - 8 hr, 15 min - 9 hr, or 15 min - 10 hr) and the like.

As the method of "heating", for example, heating with a steamer, heating with a microwave oven, heating with an oven, and the like can be mentioned.

The "heating" includes modes of heating or reheating cheese, or cooked or semi-cooked dishes containing cheese. Specific examples include, but are not limited to, a mode of steam-heating steamed buns and steamed bread containing cheese in the filling at a convenience store or the like with a steamer, a mode of heating a frozen food containing cheese, and a mode of reheating a prepared side dish containing cheese in a microwave oven or the like.

In the present specification, the effect of suppressing "decrease in texture and/or stretchability of cheese due to heating" can be evaluated, for example, by the method of the below-mentioned Experimental Example 1 or 2 or a method analogous thereto.

In the present invention, the "cheese texture" refers to the softness and smoothness on the tongue when eaten.

In the present invention, "cheese stretchability" refers to the property of being stretched without breaking when physically separated. In the present specification, the "elasticity" refers to the property of trying to return to its original shape when physically separated. Since cheese preferably has elasticity in addition to high stretchability to achieve good texture, both stretchability and elasticity were evaluated in the present invention.

The present invention relates to a method for suppressing a decrease in texture and/or stretchability of cheese due to heating, including allowing an enzyme of any of enzymes (1) to (7) to act on raw cheese.

In the method for suppressing a decrease in texture and/or stretchability of cheese due to heating of the present invention, the amounts of enzymes (1) to (7) to be added, the ratio of the amounts to be added, and the method of addition are the same as those described with respect to the above-mentioned production method of cheese that shows a suppressed decrease in texture and/or stretchability of cheese due to heating.

The present invention relates to an enzyme preparation for suppressing a decrease in texture and/or stretchability of cheese due to heating, which contains enzymes (1) to (7) and is allowed to act on raw cheese.

In the enzyme preparation of the present invention, the content ratio of the enzymes is the same as that described with respect to the above-mentioned production method of cheese that shows a suppressed decrease in texture and/or stretchability of cheese due to heating.

The enzyme preparation of the present invention can suppress a decrease in texture and/or stretchability of cheese due to heating, by adding the enzyme preparation to raw cheese to cause reaction, according to the method described with respect to the above-mentioned production method of cheese that shows a suppressed decrease in texture and/or stretchability of cheese due to heating.

The enzyme preparation of the present invention may further contain, in addition to enzymes (1) to (7), excipients such as dextrin, indigestible dextrin, hydrogenated maltose, and the like, seasonings such as meat extract and the like, proteins such as plant protein, gluten, egg white, gelatin, casein, and the like, protein hydrolysate, protein partial hydrolysate, emulsifier, chelating agents such as citrate, polyphosphosphate, and the like, reducing agents such as glutathione, cysteine, and the like, and other food additives such as alginic acid, brine water, fat, dye, sourness, flavor, and the like. The enzyme preparation of the present invention may be in any form such as liquid, paste, granule, or powder.

The cheese produced by the production method of the present invention can be used as it is or as a food product obtained by combining with other food materials or general food products.

In the present specification, food is a concept that broadly includes foods that can be ingested orally (excluding pharmaceutical products), and includes not only so-called "food" but also beverages, health supplement, food with health claims (e.g., food for specified health uses, food with functional claims, food with nutrient function claims), supplement, and the like.

### 1. The second invention

The present invention (the second invention) is explained in detail below.

The production method of cheese analogues improved in meltability and/or stretchability during heating in the present invention (the second invention) is characterized in that it contains
(1) a step of allowing α-glucosidase to act on a mixture of starch and a plant-derived oil, or
(2) a step of allowing α-glucosidase to act on starch, mixing the obtained reaction product with a plant-derived oil.

The "starch" to be used in the present invention refers to plant-derived raw starch and modified starch generally used for food products.

Examples of the starch in the present invention include rice starch, sago starch, waxy corn starch, tapioca starch, regular corn starch, potato starch, wheat starch, hydroxypropyl distarch phosphate, acetylated distarch adipate, acetylated distarch phosphate, acetylated oxidized starch, starch sodium octenylsuccinate, starch acetate, oxidized starch, hydroxypropyl starch, phosphated distarch phosphate, monostarch phosphate, and distarch phosphate, and waxy corn starch and tapioca starch are preferred. Only one kind of starch, or two or more kinds thereof may be used in combination.

In the production method of the present invention, the amount of starch to be used is, for example, not less than 2.5 wt%, preferably not less than 5 wt%, more preferably not less than 7.5 wt%, further preferably not less than 10 wt%, with respect to a cheese analogue.

In the production method of the present invention, the amount of starch to be used is, for example, not more than 70 wt%, preferably not more than 60 wt%, more preferably not more than 50 wt%, further preferably not more than 40 wt%, with respect to a cheese analogue.

In the production method of the present invention, the amount of starch to be used is, for example, 2.5 - 70 wt%, preferably 5 - 60 wt%, more preferably 7.5 - 50 wt%, further preferably 10 - 40 wt%, with respect to a cheese analogue.

In the production method of the present invention, by setting the amount of starch to be used to fall within the above-mentioned ranges, an effect of imparting milk cheese-like smooth texture and shape retention can be obtained.

In the production method of the present invention, when the amount of starch to be used is less than 2.5 wt% with respect to a cheese analogue, the cheese analogue tends to show insufficient shape retention and become excessively soft and unpalatable.

In the production method of the present invention, when the amount of starch to be used exceeds 70 wt% with respect to a cheese analogue, the cheese analogue tends to show a crumbly texture and become unpalatable.

The "plant-derived oil" used in the present invention refers to a plant-derived oil that is generally used for food products.

The plant-derived oil in the present invention includes, for example, coconut oil, palm oil, rape seed oil, soybean oil, corn oil, safflower oil, cacao oil, and the like. Among these, coconut oil is preferred since it has a relatively high melting point, solidifies at room temperature, and imparts shape retention to cheese analogues. Only one kind of plant-derived oil may be used or two or more kinds thereof may be used in combination.

In the production method of the present invention, the amount of the plant-derived oil to be used is, for example, not less than 2.5 wt%, preferably not less than 5 wt%, more preferably not less than 7.5 wt%, further preferably not less than 10 wt%, with respect to a cheese analogue.

In the production method of the present invention, the amount of the plant-derived oil to be used is, for example, not more than 70 wt%, preferably not more than 60 wt%, more preferably not more than 50 wt%, further preferably not more than 40 wt%, with respect to a cheese analogue.

In the production method of the present invention, the amount of the plant-derived oil to be used is, for example, 2.5 - 70 wt%, preferably 5 - 60 wt%, more preferably 7.5 - 50 wt%, further preferably 10 - 40 wt%, with respect to a cheese analogue.

In the production method of the present invention, when the amount of plant-derived oil to be used is less than 2.5 wt% with respect to a cheese analogue, the cheese analogue tends to show a crumbly texture and become unpalatable.

In the production method of the present invention, when the amount of plant-derived oil to be used exceeds 70 wt% with respect to a cheese analogue, the cheese analogue tends to show insufficient shape retention and become excessively soft and unpalatable.

In the present invention, the weight ratio of starch and plant-derived oil (starch:plant-derived oil) is, for example, 1:5 - 5:1, preferably 1:4 - 4:1, more preferably 1:3 - 3:1, further preferably 1:2 - 2:1.

The α-glucosidase (EC 3.2.1.20) to be used in the present invention is an enzyme that produces α-glucose by hydrolyzing a nonreducing terminal α-1,4-glucosidic bond. Among such α-glucosidases, transglucosidase is preferred. Incidentally, an enzyme commercially available from Amano Enzyme, Inc. under a trade name of "transglucosidase "Amano"", "α-glucosidase "Amano"" is one example of the α-glucosidase.

With respect to the enzyme activity of the α-glucosidase, the amount of the enzyme which produces 1 µg of glucose in 2.5 mL of a reaction mixture was defined as 1 U (unit) when 1 mL of 0.02 M acetate buffer (pH 5.0) was added to 1 mL of 1 mM α-methyl-D-glucoside, and 0.5 mL of an enzyme solution was added thereto to act at 40°C for 60 minutes.

In the present invention, the amount of α-glucosidase to be added is such that the enzyme activity per 1 g of starch is preferably 0.00001 - 10000 U, more preferably 0.0001 - 1000 U, further preferably 0.001 - 1000 U, particularly preferably 0.01 - 100 U.

In the production method of the present invention, moreover, raw materials generally used for food products may also be used in combination.

The method for producing the cheese analogue of the present invention preferably includes the following steps:
(i) a step of emulsifying by mixing plant-derived oil, starch, optionally added vegetable protein and additives (e.g., polysaccharide thickeners, additives generally used in the food product field, which will be described later), and α-glucosidase to obtain an emulsified mixture,
(ii) a step of pouring the emulsified mixture obtained in step (i) into a mold, then heating the mold without stirring to allow α-glucosidase to act on the plant-derived oil and starch in the mixture (enzyme reaction step),
(iii) a step of cooling the mixture still in the mold after completion of heating in step (ii) to obtain a cheese analogue.

In another embodiment, the method for producing the cheese analogue of the present invention preferably includes the following steps:
(i') a step of adding α-glucosidase to starch, heating the mixture to allow α-glucosidase to act on starch (enzyme reaction step), emulsifying by mixing the obtained reaction product, plant-derived oil, and optionally added vegetable protein and additives (e.g., polysaccharide thickeners, additives generally used in the food product field, which will be described later) to obtain an emulsified mixture,
(ii') a step of pouring the emulsified mixture obtained in step (I') into a mold, and then cooling the mixture still in the mold without stirring to obtain a cheese analogue.

The mixing in steps (i) and (I') can be performed by a method known in the food product manufacturing field. For example, mixing using a mixer used for production of cheese, such as food processor, cooker type emulsification equipment, kettle type emulsification equipment, vertical high-speed shearing emulsification equipment, scratping heat exchanger, and the like can be mentioned. The temperature of the mixing step is, for example, 10 - 65°C. The time for the mixing step can be appropriately selected from the time required for emulsifying the mixture.

In the production method of the present invention, the reaction time of α-glucosidase with the mixture containing plant-derived oil and starch (enzyme reaction step of the above-mentioned step (ii)), and the reaction time of α-glucosidase with starch (enzyme reaction step of the above-mentioned step (i')) are not particularly limited as long as the enzyme can act on the substrate substances, plant-derived oil, starch (the above-mentioned step (ii)), or starch (the above-mentioned step (i')). As a realistic action time, 0.015 to 24 hr is preferred. Also, the reaction temperature of α-glucosidase (enzyme reaction step in the above-mentioned step (ii) and enzyme reaction step in the above-mentioned step (i')) is not particularly limited as long as the enzyme maintains its activity. As a realistic temperature, action at 5 to 60°C is preferred.

The enzyme reaction in step (ii) and the enzyme reaction in step (i') can be completed by heating at, for example, 70 - 100°C for 10 - 120 min (enzyme deactivation step).

In the present invention, the mixture containing plant-derived oil and starch (mixture in the above-mentioned step (i)) to be acted on by α-glucosidase, and the component to be mixed with starch acted on by α-glucosidase (plant-derived oil in the above-mentioned step (i')) preferably further contain polysaccharide thickeners (e.g., gum arabic, xanthan gum, tamarind seed gum, guargum, locust bean gum, carrageenan, etc.). Only one kind of polysaccharide thickener may be used or two or more kinds thereof may be used in combination.

The amount of the polysaccharide thickener to be added is, for example, 0.001 - 60 wt%, preferably 0.01 - 50 wt%, more preferably 0.1 - 40 wt%, further preferably 1 - 30 wt%, with respect to a cheese analogue.

In the present invention, emulsifiability can be imparted to cheese analogues by containing a polysaccharide thickener.

In the present invention, the mixture containing plant-derived oil and starch (mixture in the above-mentioned step (i)) to be acted on by α-glucosidase, and the component to be mixed with starch acted on by α-glucosidase (plant-derived oil in the above-mentioned step (i')) may further contain vegetable protein (e.g., soy protein, pea protein, chickpea protein, fava bean protein, almond protein, oat protein, chia seed protein, rape seed protein, duckweed protein, microorganism-derived protein, fungi-derived protein, etc.), Only one kind of polysaccharide thickener may be used or two or more kinds thereof may be used in combination.

The amount of the vegetable protein to be added is, for example, 0.1 - 50 wt%, preferably 0.2 - 40 wt%, more preferably 0.5 - 30 wt%, further preferably 1 - 20 wt%, with respect to a cheese analogue.

In the present invention, the mixture containing plant-derived oil and starch to be acted on by α-glucosidase, or a mixture of starch acted on by α-glucosidase and plant-derived oil and the like contains water.

The amount of water to be added is, for example, 5 - 80 wt%, preferably 15 - 70 wt%, more preferably 25 - 60 wt%, further preferably 35 - 50 wt%, with respect to a cheese analogue.

In the present invention, the cheese analogues may contain additives generally used in the food product field, that are other than the above-mentioned components.

Examples of the additive include flavor (e.g., Cheddar flavor (powder, liquid), Parmesan flavor (powder, liquid), Camembert flavor (powder, liquid), cream cheese flavor (powder, liquid)), seasoning (e.g., salt, yeast extract), colorant, excipient (dextrin, lactose), various amino acids, lactic acid, plant protein, and the like.

The amount of the additive to be used is, for example, 1 - 30 wt% with respect to a cheese analogue.

By the above-mentioned production method of the present invention, cheese analogues improved in meltability and/or stretchability during heating can be produced.

In the present invention, the "heating" of the "meltability and/or stretchability during heating" refers to, for example, heating in order to melt and/or stretch the cheese analog during cooking and food product processing using cheese analogue. The temperature of heating is, for example, 70 - 200°C, and the time of the heating is, for example, 1 - 20 min. "during heating" refers to immediately after (e.g., within 20 min) completion of heating.

In the present invention, the "meltability" refers to the property of the cheese analogue to liquefy and spread.

In the present invention, the "stretchability" refers to the property of the cheese analogue to stretch like a string.

In the present invention, the "improved meltability during heating" means that the meltability during heating of the cheese analogue produced by adding the enzyme in the present invention is improved as compared with the meltability during heating of the cheese analogue produced without the addition of the enzyme.

In the present invention, the "improved stretchability during heating" means that the stretchability during heating of the cheese analogue produced by adding the enzyme in the present invention is improved as compared with the stretchability during heating of the cheese analogue produced without the addition of the enzyme.

In the present specification, the effect of improving "meltability and/or stretchability during heating" can be evaluated, for example, by the method of the below-mentioned Experimental Example 3 or a method analogous thereto.

The present invention relates to a method for improving meltability and/or stretchability of a cheese analogue during heating, comprising, during production of the cheese analogue,
(1) a step of allowing α-glucosidase to act on a mixture of starch and a plant-derived oil, or
(2) a step of allowing α-glucosidase to act on starch, mixing the obtained reaction product with a plant-derived oil.

In the method for improving meltability and/or stretchability of a cheese analogue during heating of the present invention, the definition, examples, and amount of use of starch, the definition, examples, and amount of use of plant-derived oil, the definition, amount of addition, and method of addition of α-glucosidase, components to be added optionally, additives to be added optionally, and the like are the same as the definition, examples, and amount of use of starch, the definition, examples, and amount of use of plant-derived oil, the definition, amount of addition, and method of addition of α-glucosidase, components to be added optionally, additives to be added optionally, and the like described with respect to the above-mentioned production method of the cheese analogue improved in meltability and/or stretchability during heating of the present invention.

The present invention relates to an enzyme preparation for improving meltability and/or stretchability of a cheese analogue during heating, comprising
(1) α-glucosidase to be allowed to act on a mixture of starch and a plant-derived oil during production of the cheese analogue, or
(2) α-glucosidase to be allowed to act on starch before mixing with a plant-derived oil during production of the cheese analogue.

In the enzyme preparation of the present invention, the definition, examples, and amount of use of starch, the definition, examples, and amount of use of plant-derived oil, the definition, amount of addition, and method of addition of α-glucosidase, components to be added optionally, additives to be added optionally, and the like are the same as the definition, examples, and amount of use of starch, the definition, examples, and amount of use of plant-derived oil, the definition, amount of addition, and method of addition of α-glucosidase, components to be added optionally, additives to be added optionally, and the like described with respect to the above-mentioned production method of the cheese analogue improved in meltability and/or stretchability during heating of the present invention.

The enzyme preparation of the present invention can improve the meltability and/or stretchability of cheese analogue during heating by adding the enzyme preparation to cause a reaction with a mixture containing plant-derived oil and starch, or starch before mixing with plant-derived oil, according to the addition method and amount to be added of α-glucosidase described in the above-mentioned production method of cheese analogues improved in meltability and/or stretchability during heating in the present invention.

The enzyme preparation of the present invention may further contain, in addition to α-glucosidase, excipients such as dextrin, indigestible dextrin, hydrogenated maltose, and the like, seasonings such as meat extract and the like, proteins such as plant protein, gluten, egg white, gelatin, casein, and the like, protein hydrolysate, protein partial hydrolysate, emulsifier, chelating agents such as citrate, polyphosphosphate, and the like, reducing agents such as glutathione, cysteine, and the like, and other food additives such as alginic acid, brine water, fat, dye, sourness, flavor, and the like. The enzyme preparation of the present invention may be in any form such as liquid, paste, granule, or powder.

The cheese analogue produced by the production method of the present invention can be used as it is or as a food product obtained by combining with other food materials or general food products.

In the present specification, food is a concept that broadly includes foods that can be ingested orally (excluding pharmaceutical products), and includes not only so-called "food" but also beverages, health supplement, food with health claims (e.g., food for specified health uses, food with functional claims, food with nutrient function claims), supplement, and the like.

### [Example]

The present invention is explained in more detail in the following by referring to Examples and Experimental Examples, which are not to be construed as limitative.

### [Experimental Example 1]

### (Production of cheeses of Examples 1 to 7, Comparative Example 1)

According to the blending amounts shown in Table 1, Mozzarella cheese (trade name "Mozzarella melting cheese", manufactured by QBB) and Gouda & Cheddar cheese (trade name "NC shredded cheese RKB" manufactured by QBB) were mixed for 2 minutes and 30 seconds using a food processor. Furthermore, each enzyme shown in Table 1 was added and mixed with a food processor for 1 min. The obtained mixture was placed in a standing pouch and allowed to rest in a refrigerator (5°C) for 1 hr. The mixture was further allowed to rest in a proof box (45°C) for 30 min. The obtained mixture was steamed in a steam convection oven (100°C) for 15 min to yield the cheeses of Examples 1 to 7. A cheese of Comparative Example 1 (control) was obtained in the same manner as above except that no enzyme was added. Each obtained cheese was cooled and refrigerated.

The trade name, the number of units per gram, and the like of each enzyme in Table 1 are shown in Table 2.

**[Table 1]**

| | blended amount (g) | | | | | | | |
|---|---|---|---|---|---|---|---|---|
| raw material | Comparative Example 1 | Example 1 | Example 2 | Example 3 | Example 4 | Example 5 | Example 6 | Example 7 |
| mozzarella cheese | 70 | 70 | 70 | 70 | 70 | 70 | 70 | 70 |
| Gouda & Cheddar cheese | 30 | 30 | 30 | 30 | 30 | 30 | 30 | 30 |
| glucose oxidase | - | 0.034 | - | - | 0.034 | 0.034 | - | 0.034 |
| α-glucosidase | - | - | 0.0045 | - | 0.0045 | - | 0.0045 | 0.0045 |
| transglutaminase | - | - | - | 0.007 | - | 0.007 | 0.007 | 0.007 |
| total | 100 | 100.034 | 100.0045 | 100.007 | 100.0385 | 100.041 | 100.0115 | 100.0455 |

**[Table 2]**

| enzyme name | trade name | manufacturer | unit number per 1 g (U/g) | blended amount | |
|---|---|---|---|---|---|
| | | | | (g) | (U) |
| glucose oxidase | Sumizyme PGO | SHIN NIHON CHEMICAL CO., LTD. | 2150 | 0.034 | 73.1 |
| α-glucosidase | transglucosidase "Amano" | Amano Enzyme Inc. | 608000 | 0.0045 | 2736 |
| transglutaminase | Activa TG | Ajinomoto Co., Inc. | 1150 | 0.007 | 8.05 |

The above-mentioned refrigerated cheeses of Examples 1 to 7 and Comparative Example 1 were steam-heated again in a steam convection oven (80°C) for 20 min. Immediately after completion of heating, sensory evaluation and stretchability evaluation were performed by the methods described below.

### (Sensory evaluation)

A sensory evaluation was conducted by four panelists, and the cheeses of Examples 1 to 7 and Comparative Example 1 were evaluated for softness and smoothness on tongue when eaten immediately after completion of heating. According to the definitions in Table 3 below, the evaluation was made on a scale of 1 to 5 points in 0.5 point increments, with 3 points being equivalent to Comparative Example 1 (no enzyme added, control). The score was determined by a consensus of the four panelists. The results are shown in Table 4.

### (Evaluation of stretchability of cheese)

The cheeses obtained above in Examples 1 to 7 and Comparative Example 1 were subjected to the evaluation of stretchability during heating according to the methods (1) and (2) shown below.
(1) The cheeses of Examples 1 to 7 and Comparative Example 1 were evaluated for the appearance and feel when stretched by hand. According to the definitions in Table 3 above, the evaluation was made on a scale of 1 to 5 points in 0.5 point increments, with 3 points being equivalent to Comparative Example 1 (no enzyme added, control). The score was determined by a consensus of the four panelists. The results are shown in Table 4.
(2) The cheeses (100 g) of Examples 1 to 7 and Comparative Example 1 were scooped with a fork and stretched upwards immediately after completion of heating, and the maximum length of the cheeses at which the cheeses did not break was measured. The results are shown in Table 5.

**[Table 3]**

| evaluation | 1← definitions →5 |
|---|---|
| softness | hard ← equivalent to control → soft |
| smoothness on tongue | crumbly ← equivalent to control → smooth |
| stretchability | poor stretch, chopped ← equivalent to control → good stretch, no break |

**[Table 4]**

| | score | | | comments |
|---|---|---|---|---|
| | softness | smoothness on tongue | stretchability | |
| Comparative Example 1 (no addition of enzyme) | 3.0 | 3.0 | 3.0 | faint texture, with breaks, easily torn |
| Example 1 | 4.0 | 3.5 | 3.5 | elastic, no breaks |
| Example 2 | 4.0 | 3.0 | 4.0 | weak elasticity, soft and stretchy, loose texture |
| Example 3 | 3.0 | 3.5 | 3.5 | strong elasticity, stretched slightly more than Comparative Example |
| Example 4 | 4.5 | 4.5 | 4.0 | elastic but smooth, soft and moderately stretched |
| Example 5 | 4.0 | 3.5 | 4.0 | elastic and moderately stretched |
| Example 6 | 4.0 | 3.0 | 3.5 | slightly elastic but stretched softly |
| Example 7 | 4.0 | 4.0 | 4.5 | elastic and stretches well without breaks |

**[Table 5]**

| | stretchability (cm) |
|---|---|
| Comparative Example 1 (no addition of enzyme) | 55 |
| Example 1 | 90 |
| Example 2 | 95 |
| Example 3 | 80 |
| Example 4 | 110 |
| Example 5 | 105 |
| Example 6 | 75 |
| Example 7 | 120 |

In the results of Table 4 and Table 5, the cheeses of Examples 1 to 7 obtained by respectively allowing "glucose oxidase", "a-glucosidase", "transglutaminase", "glucose oxidase and α-glucosidase", "glucose oxidase and transglutaminase", "α-glucosidase and transglutaminase", and "glucose oxidase and α-glucosidase and transglutaminase" to act on raw cheeses received high evaluation in texture (softness and/or smoothness on tongue) and stretchability, as compared with Comparative Example 1 (no addition of enzyme, control).

The results show that the cheeses acted on by "glucose oxidase", "a-glucosidase", "transglutaminase", "glucose oxidase and α-glucosidase", "glucose oxidase and transglutaminase", "α-glucosidase and transglutaminase", or "glucose oxidase and α-glucosidase and transglutaminase" suppressed a decrease in texture (softness and/or smoothness on tongue) and stretchability due to heating (steam heating) as compared with cheese without addition of enzyme (i.e., conventional cheese).

### [Experimental Example 2]

### (Production of cheeses of Examples 8 to 12, Comparative Example 2)

According to the blending amounts shown in Table 6, Mozzarella cheese (trade name "Mozzarella melting cheese", manufactured by QBB), Gouda & Cheddar cheese (trade name "NC shredded cheese RKB" manufactured by QBB), and hydroxypropyl distarch phosphate (trade name "MATSUTANI ASAGAO", manufactured by Matsutani Chemical Industry Co., Ltd.) were mixed for 2 minutes and 30 seconds using a food processor. Furthermore, each enzyme shown in Table 6 was added and mixed with a food processor for 1 min. The obtained mixture was placed in a standing pouch and allowed to rest in a refrigerator (5°C) for 1 hour. The mixture was further allowed to rest in a proof box (45°C) for 30 min. The obtained mixture was steamed in a steam convection oven (100°C) for 15 min to yield the cheeses of Examples 8 to 12. A cheese of Comparative Example 2 (control) was obtained in the same manner as above except that no enzyme was added. Each obtained cheese was cooled and refrigerated.

The trade name, the number of units per gram, and the like of each enzyme in Table 6 are the same as those in the above-mentioned Table 2.

**[Table 6]**

| | blended amount (g) | | | | | |
|---|---|---|---|---|---|---|
| raw material | Comparative Example 2 | Example 8 | Example 9 | Example 10 | Example 11 | Example 12 |
| mozzarella cheese | 70 | 70 | 70 | 70 | 70 | 70 |
| Gouda & Cheddar cheese | 30 | 30 | 30 | 30 | 30 | 30 |
| hydroxypropyl distarch phosphate | 1 | 1 | 1 | 1 | 1 | 1 |
| glucose oxidase | - | 0.034 | - | 0.034 | - | 0.034 |
| α-glucosidase | - | - | 0.0045 | 0.0045 | 0.0045 | 0.0045 |
| transglutaminase | - | - | - | - | 0.007 | 0.007 |
| total | 101 | 101.034 | 101.0045 | 101.0385 | 101.0115 | 101.0455 |

The above-mentioned refrigerated cheeses of Examples 8 to 12 and Comparative Example 2 were steam-heated again in a steam convection oven (80°C) for 20 min. Immediately after completion of heating, sensory evaluation and stretchability evaluation were performed by the methods described below.

### (Sensory evaluation)

A sensory evaluation was conducted by four panelists, and the cheeses of Examples 8 to 12 and Comparative Example 2 were evaluated for softness and smoothness on tongue when eaten, immediately after completion of heating. According to the definitions in the above-mentioned Table 3, the evaluation was made on a scale of 1 to 5 points in 0.5 point increments, with 3 points being equivalent to Comparative Example 2 (no enzyme added, control). The score was determined by a consensus of the four panelists. The results are shown in Table 7.

### (Evaluation of stretchability of cheese)

The cheeses (100 g) of Examples 8 to 12 and Comparative Example 2 were evaluated, immediately after completion of heating, for the appearance and feel when stretched by hand. According to the definitions in Table 3 above, the evaluation was made on a scale of 1 to 5 points in 0.5 point increments, with 3 points being equivalent to Comparative Example 2 (no enzyme added, control). The score was determined by a consensus of the four panelists. The results are shown in Table 7.

**[Table 7]**

| | score | | | comments |
|---|---|---|---|---|
| | softness | smoothness on tongue | stretchability | |
| Comparative Example 2 (no addition) | 3 | 3 | 3 | with breaks, easily torn |
| Example 8 | 3.5 | 3.5 | 4.5 | elastic, no breaks, stretchy |
| Example 9 | 5 | 4 | 3.5 | softened, loose texture, weak elasticity but slightly stretched |
| Example 10 | 4.5 | 4 | 4.5 | elastic but smooth, soft and stretchy |
| Example 11 | 3 | 4 | 4 | slightly elastic and stretched well |
| Example 12 | 3.5 | 5 | 5 | elastic, no breaks, stretched well |

In the results of Table 7, the cheeses of Examples 8 to 12 obtained by respectively allowing "glucose oxidase", "α-glucosidase", "glucose oxidase and α-glucosidase", "α-glucosidase and transglutaminase", and "glucose oxidase and α-glucosidase and transglutaminase" to act on raw cheeses (with hydroxypropyl distarch phosphate added) received high evaluation in texture (softness and/or smoothness on tongue) and stretchability, as compared with Comparative Example 2 (no addition of enzyme, control).

The results show that the cheeses acted on by "glucose oxidase", "α-glucosidase", "glucose oxidase and α-glucosidase", "a-glucosidase and transglutaminase", or "glucose oxidase and α-glucosidase and transglutaminase" suppressed a decrease in texture (softness and/or smoothness on tongue) and stretchability due to heating (steam heating) as compared with cheese without addition of enzyme (i.e., conventional cheese).

### [Experimental Example 3]

### (Example, Comparative Example cheese analogue production)

According to the blending rate shown in Table 8, the raw materials were emulsified by mixing for 1 min using a food processor (Bamix, manufactured by CHERRY TERRACE Inc.). The obtained mixture was poured into a mold and heated in a constant temperature water bath at 50°C for 30 min without stirring (enzyme reaction step), successively heated at 95°C for 30 min without stirring (enzyme deactivation step), and cooled in a refrigerator (5°C) for 48 hr to obtain the cheese analogues of Example and Comparative Example.

**[Table 8]**

| raw material | blending rate (wt%) | |
|---|---|---|
| | Comparative Example (no addition of α-glucosidase) | Example (a-glucosidase added) |
| waxy corn starch | 13.30 | 13.30 |
| tapioca starch | 13.30 | 13.30 |
| gum arabic | 5.00 | 5.00 |
| yeast extract | 1.20 | 1.20 |
| coconut oil | 20.00 | 20.00 |
| water | 45.60 | 45.60 |
| salt | 1.40 | 1.40 |
| lactic acid | 0.10 | 0.10 |
| colorant | 0.10 | 0.10 |
| total | 100.00 | 100.00 |
| α-glucosidase (*1) | 0 | 0.20 (*2) |

| | | |
|---|---|---|
| (*1) α-glucosidase: α-glucosidase "Amano" (trade name) (bulk powder activity 120 u/g), manufactured by Amano Enzyme Inc., (*2) The amount "0.20" of α-glucosidase means that α-glucosidase is 0.20 parts by weight with respect to a total 100.00 parts by weight of raw materials other than α-glucosidase. The enzyme activity with respect to 1 g cheese analogue in Example is 0.24 U, and the enzyme activity with respect to 1 g starch in the cheese analogue of Example is 0.9 U. | | |

### (Evaluation of meltability and stretchability of cheese analogue during heating)

The cheese analogues obtained above in Example and Comparative Example were subjected to the evaluation of meltability and stretchability of the cheese analogues during heating, according to the method shown below.

The cheese analogues (weight: 3 g, size: 20 mm×20 mm×5 mm) of Example and Comparative Example were heated in an oven at 180°C for 10 min.

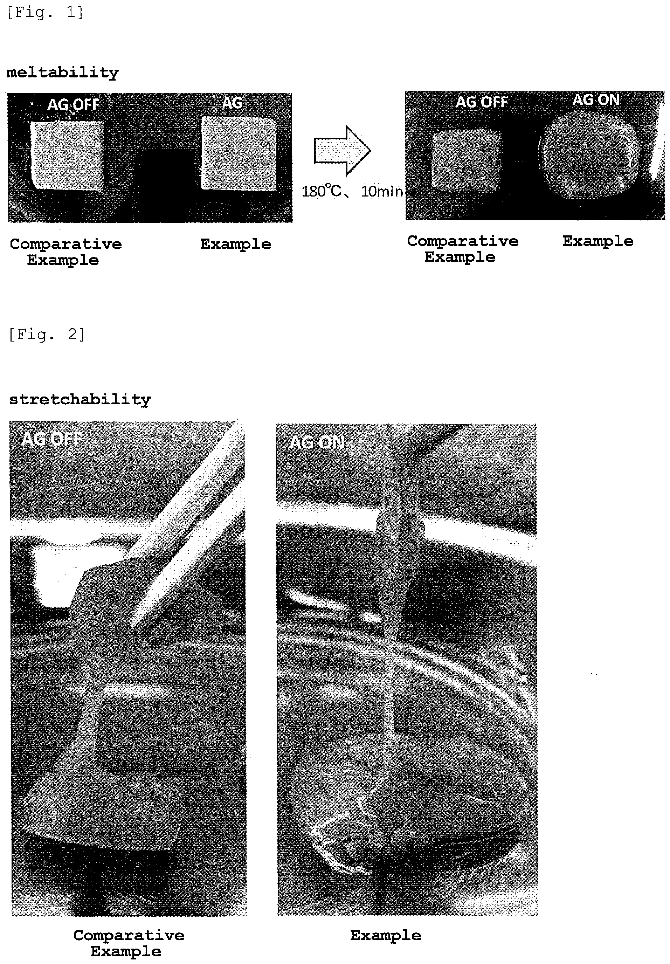

After completion of the heating, the cheese analogues of Example and Comparative Example were visually observed to evaluate meltability. The photographs of the cheese analogues of Example and Comparative Example before and after heating are shown in Fig. 1 (left photograph: before heating, right photograph: after heating).

In addition, immediately after completion of the heating, the cheese analogues of Example and Comparative Example were pinched with chopsticks and stretched upward, and the maximum length at which the cheese analogue was not cut was observed to evaluate stretchability. The photographs taken when the cheese analogues of Example and Comparative Example were stretched to the maximum length free of breakage are shown in Fig. 2.

The results in Fig. 1 show that the cheese analogue produced by allowing α-glucosidase to act thereon has good meltability as compared with the cheese analogue without addition of enzyme (that is, the conventional cheese analogues), and that the meltability during heating was improved by allowing α-glucosidase to act thereon.

The results in Fig. 2 show that the cheese analogue produced by allowing α-glucosidase to act thereon has good stretchability as compared with the cheese analogue without addition of enzyme (that is, the conventional cheese analogues), and that the stretchability during heating was improved by allowing α-glucosidase to act thereon.

### [Experimental Example 4]

### (Production of cheeses of Example 13, Comparative Example 3)

According to the blending amounts shown in Table 9, Mozzarella cheese (trade name "Mozzarella melting cheese", manufactured by QBB) and Gouda & Cheddar cheese (trade name "NC shredded cheese RKB" manufactured by QBB) were mixed for 2 minutes and 30 seconds using a food processor. Furthermore, each enzyme shown in Table 9 was added and mixed with a food processor for 1 min. The obtained mixture was placed in a standing pouch and allowed to rest in a refrigerator (5°C) for 1 hr. The mixture was further allowed to rest in a proof box (45°C) for 30 min. The obtained mixture was steamed in a steam convection oven (100°C) for 15 min to yield the cheeses of Example 13. A cheese of Comparative Example 3 was obtained in the same manner as above except that no enzyme was added.

Each obtained cheese was cooled and refrigerated.

### (Production of cheeses of Example 14, Comparative Example 4)

According to the blending amounts shown in Table 9, Mozzarella cheese (trade name "Mozzarella melting cheese", manufactured by QBB), Gouda & Cheddar cheese (trade name "NC shredded cheese RKB" manufactured by QBB), and hydroxypropyl distarch phosphate (trade name "MATSUTANI ASAGAO", manufactured by Matsutani Chemical Industry Co., Ltd.) were mixed for 2 minutes and 30 seconds using a food processor. Furthermore, each enzyme shown in Table 9 was added and mixed with a food processor for 1 min. The obtained mixture was placed in a standing pouch and allowed to rest in a refrigerator (5°C) for 1 hr. The mixture was further allowed to rest in a proof box (45°C) for 30 min. The obtained mixture was steamed in a steam convection oven (100°C) for 15 min to yield the cheeses of Example 14. A cheese of Comparative Example 4 was obtained in the same manner as above except that no enzyme was added.

Each obtained cheese was cooled and refrigerated.

The trade name, the number of units per gram, and the like of each enzyme in Table 9 are the same as those in the above-mentioned Table 2.

**[Table 9]**

| | blended amount (g) | | | |
|---|---|---|---|---|
| raw material | Comparative Example 3 | Example 13 | Comparative Example 4 | Example 14 |
| mozzarella cheese | 70 | 70 | 70 | 70 |
| Gouda & Cheddar cheese | 30 | 30 | 30 | 30 |
| hydroxypropyl distarch phosphate | - | - | 1 | 1 |
| glucose oxidase | - | 0.034 | - | 0.034 |
| α-glucosidase | - | 0.0045 | - | 0.0045 |
| transglutaminase | - | 0.007 | - | 0.007 |
| total | 100 | 100.0455 | 101 | 101.0455 |

The above-mentioned refrigerated cheeses of Examples 13, 14 and Comparative Examples 3, 4 were steam-heated again in a steam convection oven (80°C) for 20 min, 1 hr, 4 hr, or 8 hr. Immediately after completion of heating, sensory evaluation and stretchability evaluation were performed by the methods described below.

### (Sensory evaluation)

A sensory evaluation was conducted by two panelists, and the cheeses of Examples 13, 14, and Comparative Examples 3, 4 were evaluated for softness and smoothness on tongue when eaten, immediately after completion of heating. According to the definitions in the above-mentioned Table 3, the evaluation was made on a scale of 1 to 5 points in 0.5 point increments, with 3 points being equivalent to control, wherein, from among the above, the control was Comparative Example 3 (enzyme not added, hydroxypropyl distarch phosphate not added) including steam heating for 20 min after refrigerating. The score was determined by a consensus of the two panelists. The results are shown in Tables 10-1 to 10-4.

### (Evaluation of stretchability of cheese)

The cheeses obtained above in Examples 13, 14, and Comparative Examples 3, 4 were evaluated for the stretchability of cheese during heating, by the methods (1) and (2) shown below.
(1) The cheeses of Examples 13, 14, and Comparative Examples 3, 4 were evaluated for the appearance and feel when stretched by hand. According to the definitions in Table 3 above, the evaluation was made on a scale of 1 to 5 points in 0.5 point increments, with 3 points being equivalent to control, wherein, from among the above, the control was Comparative Example 3 (enzyme not added, hydroxypropyl distarch phosphate not added) including steam heating for 20 min after refrigerating. The score was determined by a consensus of the two panelists. The results are shown in Tables 10-1 to 10-4.
(2) The cheeses (100 g) of Examples 13, 14 and Comparative Examples 3, 4 were scooped with a fork and stretched upwards immediately after completion of heating, and the maximum length of the cheeses at which the cheeses did not break was measured. The results are shown in Tables 10-1 to 10-4.

**[Table 10-1]**

| (with steam heating for 20 min after refrigerating) | | | | | |
|---|---|---|---|---|---|
| | score | | | stretchability length (cm) | comments |
| | softness | smoothness on tongue | stretchability | | |
| Comparative Example 3 (control) | 3.0 | 3.0 | 3.0 | 55 | faint texture, easily torn, with breaks |
| Example 13 | 3.5 | 4.0 | 4.0 | 115 | no breaks, elastic, stretches well |
| Comparative Example 4 | 4.0 | 3.5 | 4.5 | 154 | soft, smooth, stretches thin |
| Example 14 | 5.0 | 5.0 | 5.0 | 220 | homogeneous and smooth, elastic, and stretches well |

**[Table 10-2]**

| (with steam heating for 1 h after refrigerating) | | | | | |
|---|---|---|---|---|---|
| | score | | | stretchability length (cm) | comments |
| | softness | smoothness on tongue | stretchability | | |
| Comparative Example 3 | 1.5 | 1.5 | 1.5 | 30 | oil separated, crumbly, easily torn |
| Example 13 | 2.5 | 2.0 | 3.0 | 55 | homogeneous, elastic, slightly stretched |
| Comparative Example 4 | 3.5 | 3.5 | 3.5 | 70 | soft, smooth, stretched thin |
| Example 14 | 4.0 | 4.0 | 4.0 | 90 | soft, smooth, elastic, slightly stretched |

**[Table 10-3]**

| (with steam heating for 4 hr after refrigerating) | | | | | |
|---|---|---|---|---|---|
| | score | | | stretchability length (cm) | comments |
| | softness | smoothness on tongue | stretchability | | |
| Comparative Example 3 | 1.0 | 1.0 | 1.5 | 25 | completely crumbly, coarse, no stretchability |
| Example 13 | 2.0 | 2.0 | 2.0 | 35 | elastic but crumbly and easily torn |
| Comparative Example 4 | 3.0 | 3.0 | 2.5 | 40 | faint texture, easily torn |
| Example 14 | 3.5 | 3.5 | 2.5 | 40 | soft, smooth, elastic. slightly stretched |

**[Table 10-4]**

| (with steam heating for 8 hr after refrigerating) | | | | | |
|---|---|---|---|---|---|
| | score | | | stretchability length (cm) | comments |
| | softness | smoothness on tongue | stretchability | | |
| Comparative Example 3 | 0.5 | 0.5 | 0.5 | 15 | crumbly, considerably hard texture, no stretchability |
| Example 13 | 1.0 | 1.5 | 1.5 | 25 | slightly elastic, but crumbly and torn |
| Comparative Example 4 | 3.0 | 2.0 | 1.0 | 20 | faint texture, no smoothness or stretchability |
| Example 14 | 3.0 | 3.0 | 2.5 | 45 | slightly soft, slightly smooth, elastic, slightly stretched |

In the results of Tables 10-1 to 10-4, the cheese of Example 13 obtained by allowing "glucose oxidase and α-glucosidase and transglutaminase" to act on raw cheese received high evaluation in texture (softness and/or smoothness on tongue) and stretchability even when steam heated for 8 hr after refrigerating, as compared with Comparative Example 3 (no addition of enzyme).

In the results of Tables 10-1 to 10-4, the cheese of Example 14 obtained by allowing "glucose oxidase and α-glucosidase and transglutaminase" to act on raw cheese (hydroxypropyl distarch phosphate added) received high evaluation in texture (softness and/or smoothness on tongue) and stretchability even when steam heated for 8 hr after refrigerating, as compared with Comparative Examples 3 and 4 (no addition of enzyme).

The results show that the cheeses acted on by "glucose oxidase and α-glucosidase and transglutaminase" suppressed a decrease in texture (softness and/or smoothness on tongue) and stretchability due to heating (steam heating) even when steam heated for 8 hr, as compared with cheese without addition of enzyme (i.e., conventional cheese).

### [Industrial Applicability]

According to the present invention, cheese that shows a suppressed decrease in texture and/or stretchability of cheese due to heating, and a production method thereof can be provided.

According to the present invention, cheese analogues improved in meltability and/or stretchability during heating, and a production method thereof can be provided.

This application is based on patent application No. 2021-022134 filed in Japan, the contents of which are encompassed in full herein.

## Claims

1. A method for producing cheese that shows a suppressed decrease in texture and/or stretchability of cheese due to heating, comprising allowing an enzyme of any of the following (1) to (7) :
(1) glucose oxidase
(2) α-glucosidase
(3) transglutaminase
(4) glucose oxidase and α-glucosidase
(5) glucose oxidase and transglutaminase
(6) α-glucosidase and transglutaminase
(7) glucose oxidase, α-glucosidase and transglutaminase to act on target cheese to be acted on by the enzyme.

2. The production method according to claim 1, wherein said enzyme is allowed to act on a mixture of starch and the target cheese to be acted on by the enzyme.

3. The production method according to claim 1 or 2, wherein the target cheese to be acted on by the enzyme is selected from the group consisting of mozzarella cheese, Gouda cheese, Cheddar cheese, and a combination of these.

4. A method for suppressing a decrease in texture and/or stretchability of cheese due to heating, comprising allowing an enzyme of any of the following (1) to (7):
(1) glucose oxidase
(2) α-glucosidase
(3) transglutaminase
(4) glucose oxidase and α-glucosidase
(5) glucose oxidase and transglutaminase
(6) α-glucosidase and transglutaminase
(7) glucose oxidase, α-glucosidase and transglutaminase to act on target cheese to be acted on by the enzyme.

5. The suppressing method according to claim 4, wherein said enzyme is allowed to act on a mixture of starch and the target cheese to be acted on by the enzyme.

6. The suppressing method according to claim 4 or 5, wherein the target cheese to be acted on by the enzyme is selected from the group consisting of mozzarella cheese, Gouda cheese, Cheddar cheese, and a combination of these.

7. An enzyme preparation for suppressing a decrease in texture and/or stretchability of cheese due to heating, comprising an enzyme of any of the following (1) to (7):
(1) glucose oxidase
(2) α-glucosidase
(3) transglutaminase
(4) glucose oxidase and α-glucosidase
(5) glucose oxidase and transglutaminase
(6) α-glucosidase and transglutaminase
(7) glucose oxidase, α-glucosidase and transglutaminase,
wherein the preparation is allowed to act on target cheese to be acted on by the enzyme.

8. The enzyme preparation according to claim 7 for use in allowing said enzyme to act on a mixture of starch and the target cheese to be acted on by the enzyme.

9. The enzyme preparation according to claim 7 or 8, wherein the target cheese to be acted on by the enzyme is selected from the group consisting of mozzarella cheese, Gouda cheese, Cheddar cheese, and a combination of these.

10. A method for producing a cheese analogue improved in meltability and/or stretchability during heating, comprising
(1) a step of allowing α-glucosidase to act on a mixture of starch and a plant-derived oil, or
(2) a step of allowing α-glucosidase to act on starch and mixing the obtained reaction product with a plant-derived oil.

11. The production method according to claim 10, wherein said (1) is a step of allowing α-glucosidase to act on a mixture of starch, a plant-derived oil, and a polysaccharide thickener, and
said (2) is a step of allowing α-glucosidase to act on starch, and mixing the obtained reaction product with a plant-derived oil and a polysaccharide thickener.

12. The production method according to claim 10, wherein said (1) is a step of allowing α-glucosidase to act on a mixture of starch, a plant-derived oil, and a vegetable protein, and said (2) is a step of allowing α-glucosidase to act on starch, and mixing the obtained reaction product with a plant-derived oil and a vegetable protein.

13. A method for improving meltability and/or stretchability of a cheese analogue during heating, comprising, during production of the cheese analogue,
(1) a step of allowing α-glucosidase to act on a mixture of starch and a plant-derived oil, or
(2) a step of allowing α-glucosidase to act on starch, and mixing the obtained reaction product with a plant-derived oil.

14. The improving method according to claim 13, wherein said (1) is a step of allowing α-glucosidase to act on a mixture of starch, a plant-derived oil, and a polysaccharide thickener, and
said (2) is a step of allowing α-glucosidase to act on starch, and mixing the obtained reaction product with a plant-derived oil and a polysaccharide thickener.

15. The improving method according to claim 13, wherein said (1) is a step of allowing α-glucosidase to act on a mixture of starch, a plant-derived oil, and a vegetable protein, and said (2) is a step of allowing α-glucosidase to act on starch, and mixing the obtained reaction product with a plant-derived oil and a vegetable protein.

16. An enzyme preparation for improving meltability and/or stretchability of a cheese analogue during heating, comprising
(1) α-glucosidase to be allowed to act on a mixture of starch and a plant-derived oil during production of the cheese analogue, or
(2) α-glucosidase to be allowed to act on starch before mixing with a plant-derived oil during production of the cheese analogue.

17. The enzyme preparation according to claim 16, wherein said (1) is α-glucosidase to be allowed to act on a mixture of starch, a plant-derived oil, and a polysaccharide thickener during production of the cheese analogue, and
said (2) is α-glucosidase to be allowed to act on starch before mixing a plant-derived oil and a polysaccharide thickener during production of the cheese analogue.

18. The enzyme preparation according to claim 16, wherein said (1) is α-glucosidase to be allowed to act on a mixture of starch, a plant-derived oil, and a vegetable protein during production of the cheese analogue, and
said (2) is α-glucosidase to be allowed to act on starch before mixing a plant-derived oil and a vegetable protein during production of the cheese analogue.
